# EUROPEAN PATENT APPLICATION

(11) **EP 4 497 832 A1**
(43) Date of publication of application: **29.01.2025**
(21) Application number: 23775286.0
(22) Date of filing: 22.03.2023
(51) Int. Cl.: C12Q 1/6816, C12Q 1/6834, C12Q 1/70

(54) **DIAGNOSTIC COMPLEX, RAPID POINT-OF-CARE DIAGNOSTIC KIT USING SAME, AND DIAGNOSTIC METHOD**

(30) Priority: 22.03.2022 KR 20220035255; 26.05.2022 KR 20220064906
(71) Applicant: Korea University Research and Business Foundation, Seoul 02841 (KR)
(72) Inventor: SHIN, Se-Hyun, Seoul 06629 (KR); LEE, Jae-Seung, Seoul 02841 (KR)
(74) Representative: Meissner Bolte Partnerschaft mbB
(86) International application number: PCT/KR2023/003758
(87) International publication number: WO 2023/182790

(57) **Abstract**

The present invention relates to a complex for diagnosis, which is capable of rapidly diagnosing a target substance with high sensitivity, a rapid on-site diagnostic kit using the same and a diagnostic method, and the complex for diagnosis includes a probe DNA that binds to the target substance contained in a sample, and which can discharge thousands of Au³⁺ ions when a reagent is injected, thus enabling the rapid diagnosis of the target substance with high sensitivity.

## Description

### CROSS-REFERENCE TO RELATED APPLICATIONS

This application claims priority to and the benefit of Korean Patent Application No. 10-2022-64906 filed in the Korean Intellectual Property Office on March 22, 2022 and Korean Patent Application No. 10-2022-35255 filed in the Korean Intellectual Property Office on March 22, 2023, the entire contents of which are incorporated herein by reference.

### TECHNICAL FIELD

The present invention relates to a complex for diagnosis, which is capable of rapidly diagnosing a target substance with high sensitivity, a rapid on-site diagnostic kit using the same and a diagnostic method.

### BACKGROUND ART

The existing rapid on-site diagnostic kit is based on an antigen-antibody binding reaction, and has a problem in that the detection limit is low because the spike protein of the virus is detected using an antibody as an antigen. As a result, when the virus is detected, false negatives that are actually positive but negative appear, and this may result in the infected person neglecting initial treatment without knowing the infection or spreading the infection around, thereby increasing human and material damage.

Existing commercialized rapid on-site diagnostic kit includes kits that detect target substances using color changes of gold nanoparticles. However, since the diagnostic kit using the color change of gold nanoparticles uses the color change of gold nanoparticles fixed to the surface of the nitrocellulose membrane in proportion to the number of antigens, sensitivity may be lowered when there is not enough target material.

Meanwhile, the method of directly detecting a target material is based on signal amplification using polymerase chain reaction (PCR), and is much more advantageous in terms of sensitivity. However, PCR requires dedicated equipment and cannot be used for "rapid field detection" because amplification takes time.

### SUMMARY OF THE INVENTION

The present disclosure is designed to solve the above-described problems, and therefore the present disclosure is directed to providing a complex for diagnosis capable of rapidly diagnosing a target material with high sensitivity, a rapid on-site diagnostic kit using the same and a diagnostic method.

Other objects and advantages of the present invention will be described below and will be appreciated by embodiments of the present invention. In addition, the objects and advantages of the present invention may be realized by means and combinations shown in Claims.

According to an aspect of the present invention, there is provided a diagnostic complex used in a rapid on-site diagnostic kit to move together with a sample, the diagnostic complex including: a spherical condensed ion collection nanostructure; and a probe DNA attached to a surface of the ion collection nanostructure and bound to a target material included in the sample, wherein the probe DNA includes an Au3+ ion (gold ion) and a thiol group selectively bound to the Au3+ ion.

In addition, the ion set nanostructure is characterized in that the Au3+-DNA nanostructure.

In accordance with another aspect of the present invention, there is provided a rapid on-site diagnostic kit for moving a sample in one direction to diagnose a target material in the sample, the kit including: a nitrocellulose membrane including a test line to which a capture agent reacting with the target material is fixed and a control line through which the development of the sample is confirmed; a conjugate pad formed on one side of the nitrocellulose membrane and treated with the diagnostic complex to which the target material is bound is overlapped with the conjugate pad; a sample pad for dispensing the sample is overlapped with the conjugate pad; and an absorption pad for absorbing a material of which the reaction is completed is provided on the other side of the nitrocellulose membrane.

In addition, when a target material is included in the sample sample, the sample sample may move in the diagnostic kit such that the target material included in the sample sample may specifically bind to the diagnostic complex, and the target material bound to the diagnostic complex may move and be bound to the capture agent reacting with the target material, thereby being fixed in the test line.

In addition, it is characterized in that silver nanoparticles are fixed to the test line and the control line, and when the dithiothreitol reagent is injected into the detection window, a plurality of Au3+ ions are released from the complex for diagnosis, and the released plurality of Au3+ ions react with the silver nanoparticles to cause a color change.

In addition, when the target substance is not contained in the sample sample, the sample sample does not specifically bind to the diagnostic complex.

In addition, the diagnostic method for diagnosing a target material in a sample sample using the aforementioned rapid on-site diagnostic kit is characterized by comprising the steps of: injecting the sample sample into a sample pad of the diagnostic kit; and injecting a dithiothreitol reagent into a detection window after the injected sample passes through a test line and a control line of the diagnostic kit.

In addition, in the injecting of the sample sample into the sample pad of the diagnostic kit, when the target material is included in the sample sample, the sample sample may move in the diagnostic kit so that the target material included in the sample sample may specifically bind to the diagnostic complex, and the target material bound to the diagnostic complex may move and be fixed in the test line by binding with the capture agent reacting to the target material.

In addition, it is characterized in that silver nanoparticles are fixed to the test line and the control line, and when the dithiothreitol reagent is injected into the detection window, a plurality of Au3+ ions are released from the complex for diagnosis, and the released plurality of Au3+ ions react with the silver nanoparticles to cause color change.

In the injecting of the sample sample into the sample pad of the diagnostic kit, when the target material is not included in the sample sample, the sample sample does not specifically bind to the diagnostic complex.

### BRIEF DESCRIPTION OF THE DRAWINGS

FIG. 1 is a view schematically illustrating a process of preparing a complex for diagnosis according to the present invention.
FIG. 2 is a diagram briefly illustrating a process of synthesizing an ion-aggregate nanostructure according to the present invention.
FIG. 3 is a diagram schematically illustrating a configuration of a rapid on-site diagnostic kit according to the present invention.
FIG. 4 is a diagram illustrating a result of using the rapid on-site diagnostic kit according to the present invention.

### DETAILED DESCRIPTION

Details of other embodiments are included in the detailed description and drawings.

Advantages and features of the present disclosure, and methods of achieving the advantages and features will become apparent with reference to embodiments described below in detail in conjunction with the accompanying drawings. However, the present invention is not limited to the embodiments disclosed below, but may be implemented in various different forms, and when a part is connected to another part in the following description, the part includes not only a case of being directly connected but also a case of being connected with another medium interposed therebetween. In addition, in the drawings, parts irrelevant to the present disclosure are omitted for clarity of the description of the present disclosure, and similar parts are denoted by the same reference numerals throughout the specification.

Hereinafter, the present invention will be described with reference to the accompanying drawings.

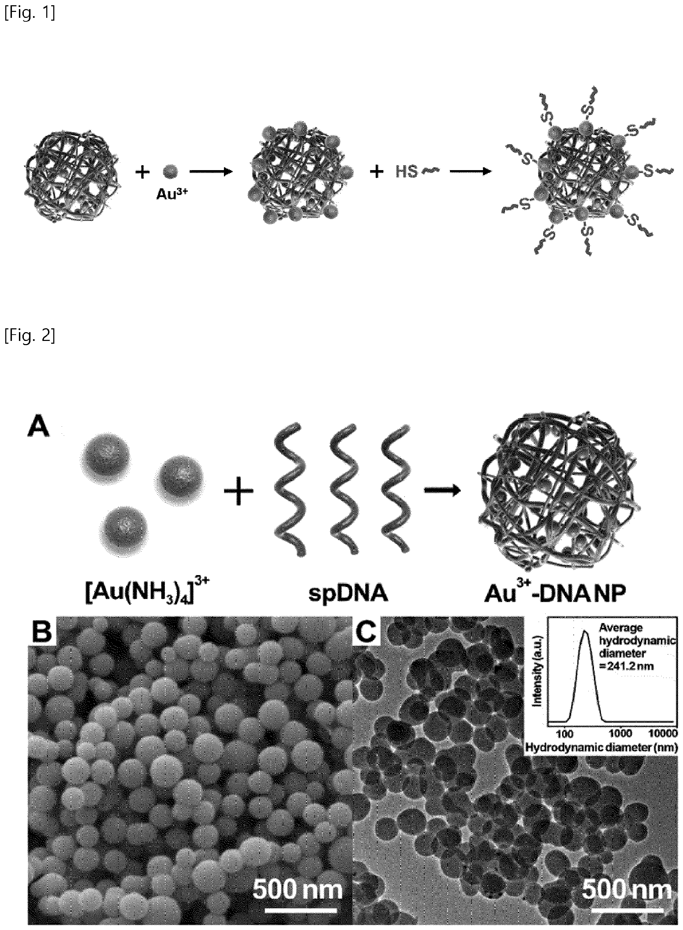

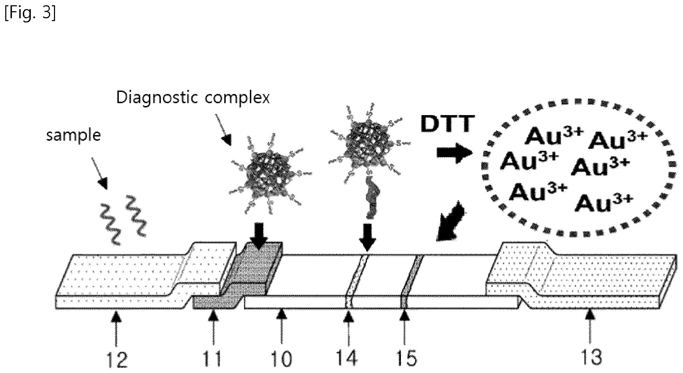

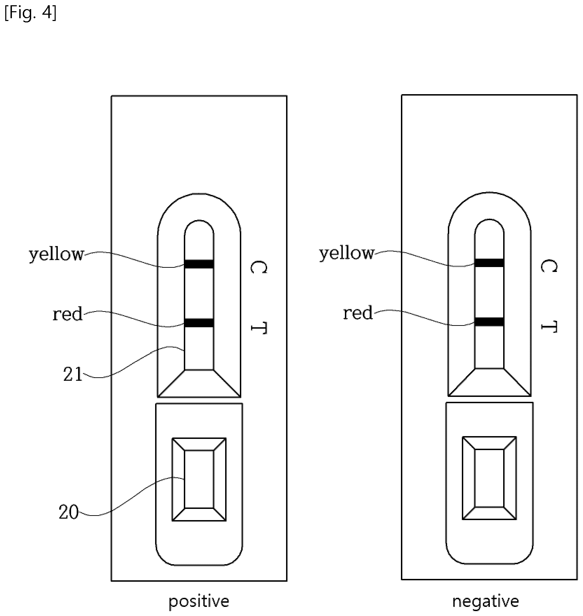

FIG. 1 is a diagram schematically illustrating a process of preparing a complex for diagnosis according to the present invention, FIG. 2 is a diagram schematically illustrating a process of synthesizing an ion set nanostructure according to the present invention, FIG. 3 is a diagram schematically illustrating a configuration of a rapid on-site diagnostic kit according to the present invention, and FIG. 4 is a diagram illustrating a result using the rapid on-site diagnostic kit according to the present invention.

### Complex for diagnosis

According to an aspect of the present invention, provided is a complex for diagnosis which is used in a rapid on-site diagnostic kit to move together with a sample.

Referring to FIG. 1, the diagnostic complex may include an ion-set nanostructure condensed in a spherical shape, and a probe DNA attached to a surface of the ion-set nanostructure and bound to a target material included in the sample.

The probe DNA may include a Au³⁺ ion and a thiol (-HS) group selectively binding to the Au³⁺ ion.

That is, the probe DNA may be obtained by attaching Au³⁺ positively charged to the negatively charged surface of the ion-set nanostructure and selectively binding a thiol group to Au3+.

The ion set nanostructure may be a Au³⁺-DNA nanostructure condensed in a spherical shape.

As shown in FIG. 2A, the ion set nanostructure was synthesized using gold complex ions, Au³⁺ cation, and salmon sperm double-stranded DNA (spDNA). At this time, [Au(NH3)4]³⁺ was used as a positive charge Au reagent.

It was confirmed that the mixture of [Au(NH3)4]³⁺ and spDNA was composed of very uniform nanoparticles (D=156 and 240 nm, respectively, according to transmission electron microscope (TEM) and dynamic light scattering (DLS) data) (see FIGS. 2B and 2C).

For reference, FIGS. 2B and 2C show SEM and TEM images of the ion-set nanostructure, respectively, and the graph inserted in FIG. 2C shows the hydrodynamic diameter distribution of the ion-set nanostructure.

### Rapid on-site diagnostic kit

According to another aspect of the present disclosure, there is provided a rapid on-site diagnostic kit that reacts with a target material by moving a sample containing the target material in one direction.

Referring to FIG. 3, the rapid on-site diagnostic kit may include a nitrocellulose membrane 10, a conjugate pad 11, a sample pad 12, and an absorption pad 13.

The nitrocellulose membrane 10 may include a test line 14 to which a capture agent reacting with the target material is fixed, and a control line 15 capable of checking the development of the sample.

A material that does not react to the target material, for example, silver nanoparticles (AgNPs), is fixed to the test line 14 and the control line 15.

The test line 14 is positioned closer to the conjugate pad 11 than the control line 15.

The capturing agent may be made of a material that specifically binds or reacts to the target material.

The conjugate pad 11 treated with the diagnostic complex that binds to the target material is formed on one side of the nitrocellulose membrane 10 to overlap.

The conjugation pad 11 refers to a pad that includes the above-described diagnostic complex and accommodates the sample moved from the sample pad 12 so that the diagnostic complex and the target substance included in the sample are bound to each other. The conjugate pad 11 may include a diagnostic complex that binds to a target substance, which is an analysis object in a sample, in a dry state. When a liquid sample is applied to the sample pad 12, the liquid sample wets the sample pad 12 in a dry state and then moves to the conjugate pad 11, so that the target substance included in the sample sample specifically binds to the diagnostic complex.

Meanwhile, a sample pad 12 for dispensing the sample may be provided on the conjugate pad 11 to overlap.

The sample pad 12 refers to a pad capable of diffusing and flowing a target material by absorbing a sample. In the present invention, the sample means a substance suspected of containing a target substance to be diagnosed, and may also be referred to as a specimen.

Preferably, the sample may include viral RNA as a target material.

The other side of the nitrocellulose membrane 10 is provided with an absorption pad 13 that absorbs the material on which the reaction has been completed.

After the injected sample passes through the test line 14 and the control line 15 of the diagnostic kit, a dithiothreitol (DTT) reagent causing the disassembly of the diagnostic complex may be additionally injected into a detection window 21 (e.g., a portion of the nitrocellulose membrane 10 including the test line 14 and the control line 15).

When the DTT reagent is injected into the detection window 21, for example, thousands of Au3+ ions are released from the diagnosis complex, and the released thousands of Au3+ ions react with silver nanoparticles (AgNPs) fixed to the test line 14 and the control line 15 to change the color.

The DTT reagent is a reagent serving as a detonator for breaking down a complex for diagnosis, and 60 million Au3+ are released from one complex for diagnosis to perform a galvanic exchange reaction with silver nanoparticles (AgNPs) (see Formula 1). As a result, the silver nanoparticles are oxidized to disappear, and gold nanoparticles (AuNPs) are formed at the positions thereof, thereby showing a color change reaction in which the yellow color of the silver nanoparticles is changed to the red color of the gold nanoparticles, and thus it may be regarded as a signal that a target material, for example, viral RNA is present.

Chemical formula 1 3*Ag*(*s*)*+Au*³⁺(*aq*)→*Au*(*s*)+3*Ag*⁺ (*aq*)

As the concentration of the target material increases, the concentration of the diagnostic complex fixed to the test line 14 increases, and as a result, the concentration of Au3+ increases, so that a change in yellow-to-red may clearly occur.

### Diagnostic method using a rapid on-site diagnostic kit

According to still another aspect of the present disclosure, there is provided a diagnostic method for diagnosing a target substance using the aforementioned rapid on-site diagnostic kit.

The diagnostic method may include injecting a sample sample containing the target material into a sample window 20 of the diagnostic kit, and injecting a DTT reagent into a detection window 21 after the injected sample sample passes through a test line 14 and a control line 15 of the diagnostic kit.

Here, in the step of injecting the sample sample containing the target material into the sample window 20 of the diagnostic kit, when the target material is contained in the sample, the sample sample moves in the diagnostic kit so that the target material contained in the sample is specifically bound to the diagnostic complex, and then the target material bound to the diagnostic complex moves and is bound to the capture agent reacting with the target material, so that the target material may be fixed in the test line 14.

As described above, when the DTT reagent is injected into the detection window 21, for example, thousands of Au3+ ions are released from the complex for diagnosis, and the released thousands of Au3+ ions move to react with the silver nanoparticles fixed to the test line 14 and the control line 15, thereby changing the color.

Referring to FIG. 4, as a result of the diagnosis, when both the control line C and the test line T are positive, the control line C and the test line T are red, and when the result of the diagnosis is negative, no reaction occurs, so that the control line C and the test line T are yellow, thereby determining whether or not the virus is infected.

Reference numeral 20 denotes a sample window through which a sample is dropped, and reference numeral 21 denotes a detection window in which a detection result is shown.

Those skilled in the art to which the present invention pertains will be able to understand that the present invention may be embodied in other specific forms without changing the technical spirit or essential features thereof. Therefore, it should be understood that the embodiments described above are exemplary and not restrictive in all aspects. The scope of the present invention is indicated by the scope of a patent claim to be described later rather than the detailed description, and it should be interpreted that all changes or modifications derived from the meaning and scope of the scope of the patent claim and the equal concept thereof are included in the scope of the present invention.

## Claims

1. A diagnostic complex that is used in a rapid on-site diagnostic kit to move along with a sample, the diagnostic complex comprising: an ion collection nanostructure that is sphericalcondensed; and a probe DNA that is attached to a surface of the ion collection nanostructure and binds to a target material included in the sample, wherein the probe DNA comprises Au³⁺ ions and a thiol group that selectively binds to the Au³⁺ ions.

2. The diagnostic complex of claim 1, wherein the ion-set nanostructure is a Au³⁺-DNA nanostructure.

3. A rapid on-site diagnostic kit for moving a sample in one direction to diagnose a target material in the sample, the kit comprising: a nitrocellulose membrane including a test line to which a capturing agent reacting to the target material is fixed and a control line through which deployment of the sample is confirmed; a conjugate pad formed by overlapping a treated complex for diagnosis, which is bound to the target material, on one side of the nitrocellulose membrane; a sample pad for loading the sample on the conjugate pad; and an absorption pad for absorbing a material which has completed reaction, on the other side of the nitrocellulose membrane.

4. The rapid on-site diagnostic kit of claim 3, wherein when a target material is included in the sample sample, the sample sample moves in the diagnostic kit so that the target material included in the sample sample specifically binds to the diagnostic complex, and the target material bound to the diagnostic complex moves and binds to a capture agent reacting to the target material to be fixed in a test line.

5. The rapid on-site diagnostic kit of claim 3, wherein silver nanoparticles are fixed to the test line and the control line, and when the dithiothreitol reagent is injected into the detection window, a plurality of Au³⁺ ions are released from the complex for diagnosis, and the released plurality of Au3+ ions react with the silver nanoparticles to cause color change.

6. The rapid on-site diagnostic kit of claim 3, wherein when the target material is not included in the sample sample, the sample sample does not specifically bind to the diagnostic complex.

7. A diagnostic method for diagnosing a target substance in a sample using the rapid on-site diagnostic kit according to claim 3, the diagnostic method comprising: injecting the sample into a sample pad of the diagnostic kit; and injecting a dithiothreitol reagent into a detection window after the injected sample passes through a test line and a control line of the diagnostic kit.

8. The diagnostic method according to claim 7, wherein in the injecting of the sample sample into the sample pad of the diagnostic kit, when a target material is contained in the sample sample, the sample sample moves in the diagnostic kit so that the target material contained in the sample sample specifically binds to the diagnostic complex, and the target material bound to the diagnostic complex moves and binds to the capture agent reacting to the target material so that the target material is fixed in the test line.

9. The diagnostic method of claim 7, wherein silver nanoparticles are fixed to the test line and the control line, and when the dithiothreitol reagent is injected into the detection window, a plurality of Au³⁺ ions are released from the complex for diagnosis, and the released plurality of Au³⁺ ions react with the silver nanoparticles to change color.

10. The diagnostic method according to claim 7, wherein in the injecting of the sample into the sample pad of the diagnostic kit, if the target material is not contained in the sample, the sample does not specifically bind to the diagnostic complex.
